# EUROPÄISCHE PATENTSCHRIFT

(11) **EP 1 552 856 B1**
(45) Veröffentlichungstag und Bekanntmachung des Hinweises auf die Patenterteilung: **07.10.2015**
(21) Anmeldenummer: 05005740.5
(22) Anmeldetag: 17.07.1998
(51) Int. Cl.: A61L 31/02, A61L 31/14, A61B 17/12

(54) **In vivo abbaubares metallisches Implantat**
Metallic implant which is degradable in vivo
Implant métallique dégradable in vivo

(30) Priorität: 18.07.1997 DE 19731021
(43) Veröffentlichungstag der Anmeldung: 13.07.2005
(62) Teilanmeldung aus: 02019905.5
(73) Patentinhaber: Biotronik AG, 8180 Bülach (CH)
(72) Erfinder: Heublein, Bernd, Prof.Dr., deceased (DE); Hausdorf, Gerd, Prof.Dr., deceased (DE)
(74) Vertreter: Randoll, Sören

(56) Entgegenhaltungen:
- US-A- 3 687 135
- ANDREW A. GAGE ET AL.: "Experimental Coronary Thrombosis in the Dog. Description of a Method", ANNALS OF SURGERY, vol. 143, no. 4, 1 April 1956 (1956-04-01) , pages 535-543,

## Beschreibung

Die vorliegende Erfindung betrifft Implantate aus metallischen Werkstoffen zum Einsatz in dem menschlichen oder tierischen Körper.

Derartige Implantate sind grundsätzlich seit langem bekannt. Die ersten Implantate wurden zu orthopädischen Zwecken entwickelt, beispielsweise Schrauben und Nägel zum Fixieren von Knochenbrüchen. Diese bestanden zunächst aus relativ einfachen Eisenlegierungen, die unter in vivo-Bedingungen zu Korrosion neigten. Die Korrosion führte dazu, dass in unmittelbarer Nähe des Knochens Metalle als Ionen freigesetzt wurden, die einen unerwünschten Anreiz für das Wachstum des Knochengewebes gegeben haben. Der Knochen ist stärker gewachsen, als es eigentlich erwünscht und erforderlich ist. Hierdurch wurde das gesunde Knochenmaterial geschädigt.

Aus diesem Grund ist man bestrebt, metallische Implantate grundsätzlich aus möglichst korrosionsbeständigen Materialien zu fertigen. Hier sind derzeit hauptsächlich korrosionsbeständige Edelstähle, Tantal und Titan im Gebrauch. Diese Implantate bleiben nach der Implantierung als Fremdkörper präsent und werden als solche vom Organismus erkannt. Sie sind nur durch eine zweite Operation zu entfernen.

Außerdem sind metallische Implantate im Bereich der Gefäßchirurgie und der Kardiologie, Angiologie und Radiologie bekannt. Diese Implantate umfassen zum Beispiel endoluminale und Gefäßstützen (Stents) zur Behandlung von Läsionen. Diese Stützen dienen z. B. zur Aufweitung und Lumenerhaltung von verengten Gefäßen, indem sie vom Gefäßlumen ausgehend mit einem Ballonkatheter (balloon expandable) oder selbstexpandierend (self expanding) das Gefäßlumen auf einem entsprechend optimalen Innendurchmesser halten. Das Implantat ist an sich nur so lange erforderlich, bis das erkrankte Gefäß durch biologische Reparaturvorgänge aus eigener Kraft den erforderlichen Durchmesser dauerhaft halten kann. Dies ist im allgemeinen etwa 4 Wochen nach Implantation der Fall.

Der dauerhafte Verbleib eines metallischen Implantats ist jedoch mit einigen Nachteilen verbunden. Das Implantat führt als Fremdkörper zu lokalen und eventuell auch systemischen Reaktionen. Zusätzlich wird die Selbstregulation des betroffenen Gefäßsegments behindert. Die ständige (pulsatile) Belastung des Metalls kann zu Ermüdungsbrüchen führen, was bei großlumigen Implantaten (z. B. Verschlusssystemen wie Schirmchen) zu neuen medizinischen Problemen führen kann. Gefäßstützen in kleineren Lumina (2,5 - 6 mm) erzeugen in etwa 20% eine erneute Stenosierung (sogenannte In-Stent-Stenose), was bei der hohen Zahl der Implantate kumulativ zu einer zusätzlichen medizinischen und ökonomischen Belastung führt. In einigen Gefäßregionen (z. B. extrakranielle Gefäße, Beinarterien) kann die metallische Struktur durch Krafteinwirkung von außen dauerhaft verformt werden mit den Folgen einer erneuten Gefäßobstruktion bzw. eines induzierten Gefäßverschlusses. Jedes Dauerimplantat ist zusätzlich mit Problemen insbesondere für jüngere Patienten deshalb verbunden, weil ein Verbleiben für Jahrzehnte unausweichlich ist.

Vollkommen biologisch abbaubare Implantate sind bislang nur aus Kunststoffmaterialien bekannt, beispielsweise aus der DE 2502884 C2. Dort wird eine Beschichtung eines orthopädischen Implantats mit Polymethylmethacrylat offenbart, das biodegradabel ist. Andere Kunststoffmaterialien umfassen Polylactid- und Polyglycolsäureester. Außerdem ist aus der EP 0006544 B1 ein biodegradables Keramikmaterial auf Basis von Calciumphosphat bekannt, das ebenfalls zur Beschichtung von metallischen Implantaten dient.

Weiterhin ist aus der WO 81/02668 ein orthopädisches Implantat bekannt, das einen korrosionsbeständigen metallischen Grundkörper sowie eine biologisch abbaubare, metallische Zwischenschicht für den Kontaktbereich zum Knochen ausweist. Diese Zwischenschicht bildet zusammen mit dem Grundkörper eine elektrochemische Zelle und erzeugt eine elektrische Spannung, die das Knochenwachstum fördert. Gleichzeitig wird die Oberflächenschicht, die beispielsweise aus Silberlegierungen bestehen kann, abgebaut. Dies führt zu dem angestrebten Effekt, dass das Knochenwachstum so lange positiv beeinflusst wird, wie es erforderlich ist und dann nach vollständigem Abbau der Oberflächenbeschichtung der elektrische Reiz nachlässt.

Die US 3 687 135 A betrifft eine magnesiumbasierte Legierung, welche als Material zum Fixieren und Verbinden von Knochenfragmenten in Form von orthopädischen Implantaten bei chirurgischen Eingriffen am Knochen Verwendung finden soll.

Schließlich betrifft die wissenschaftliche Publikation gemäß Gage et al., "Experimental Coronary Thrombosis in the Dog - Description of a Method", Annals of Surgery, Vol. 143, Nr. 4, Seiten 535 bis 543, 1956*,* ein Verfahren zur gezielten Induktion bzw. Entstehung von Thrombose im tierexperimentellen Versuch, wobei hierzu Drähte aus einer Magnesiumlegierung eingesetzt werden.

Bisher bekannte biodegradable Substanzen auf Polymerbasis werden in der Gefäßchirurgie verwendet. Ihre mechanischen Eigenschaften einerseits und die nachfolgende Fremdkörperreaktion während der Biodegradation andererseits führen dazu, dass sie als alleiniges Material für eine Implantation ungeeignet sind. Metallische Werkstoffe/Legierungen besitzen günstige mechanische Eigenschaften (Elastizität, Verformbarkeit, Stabilität) bei geringerer Masse, was für die Applikation durch dünnlumige Führungssysteme bei transkutanem Vorgehen eine wichtige Voraussetzung darstellt.

Es ist daher Aufgabe der vorliegenden Erfindung, Implantate aus biodegradablem Material zur Verfügung zu stellen, die zugleich vorteilhafte mechanische Eigenschaften aufweisen.

Diese Aufgabe wird durch Implantate mit den Merkmalen des Anspruchs 1 gelöst.

Zur Lösung des zuvor geschilderten Problems schlägt die vorliegende Erfindung ein medizinisches Implantat aus einem durch Korrosion in vivo abbaubaren metallischen Werkstoff, wobei der Werkstoff eine Legierung ist, deren Hauptbestandteil Magnesium ist, gemäß Patentanspruch 1 vor; weitere, vorteilhafte Ausgestaltungen sind Gegenstand der diesbezüglichen Unteransprüche (Patentansprüche 2 bis 13).

Weil das medizinische Implantat aus einem metallischen Werkstoff gefertigt ist, der durch Korrosion in vivo abbaubar ist, liegen primär die mechanischen Vorteile metallischer Werkstoffe vor. Der korrosive Abbau des Implantats innerhalb einer durch Materialwahl einstellbaren Zeitskala verhindert andererseits, dass die negativen Langzeiteffekte des metallischen Fremdkörpers eintreten. Dabei ist es biologisch vorteilhaft, wenn der Werkstoff eine Legierung oder ein Sintermetall ist, deren Hauptbestandteil Magnesium ist.

Die biologischen, mechanischen und chemischen Eigenschaften der Werkstoffe sind positiv beeinflussbar, wenn als Nebenbestandteil Mangan, Kobalt, Nickel, Chrom, Kupfer, Cadmium, Blei, Zinn, Thorium, Zirkonium, Silber, Gold, Palladium, Platin, Rhenium, Silicium, Calcium, Lithium, Aluminium, Zink, Eisen, Kohlenstoff oder Schwefel vorgesehen ist. Als Material wird insgesamt derzeit eine Legierung aus Magnesium mit einem Anteil von bis zu 40% Lithium sowie Eisenzusatz bevorzugt.

Vorteilhafte Abbauzeiten haben sich weiter ergeben bei Werkstoffen mit dem Hauptbestandteil Magnesium und entweder
- 0 - 40% Lithium, 0 - 5% Eisen und unter 5% andere Metalle oder seltene Erden;
- 2 - 5% Aluminium, 0 - 12% Lithium und 1 - 4% seltene Erden, insbesondere Cer, Lanthan, Neodym und/oder Praseodym,
- 6 - 12% Lithium, 2% Aluminium und 1% seltene Erden,
- 0 - 8% Lithium, 2 - 4% Aluminium und 1 - 2 % seltene Erden,
- 8,5 - 9,5% Aluminium, 0,15% - 0,4% Mangan, 0,45 - 0,9% Zink
- 4,5 - 5,3% Aluminium, 0,28% - 0,5% Mangan oder
- 30 - 40% Lithium und 0 - 5% andere Metalle und/oder seltene Erden enthält.

Derartige Magnesiumlegierungen sind z.B. unter den Bezeichnungen AZ91D, AM50A und AE42 verfügbar.

Das medizinische Implantat wird in mehreren Grundvarianten ausgeführt. Für eine Gefäßstütze ist als Grundkörper ein rohrförmiger Aufbau mit zusätzlicher Bearbeitung vorgesehen. Als Verschlusssystem (z. B. Ductus Botalli, angeborene und erworbene Septumdefekte, arterio-venöse Shuntverbindungen) sind passiv und/oder aktiv entfaltbare Schirmformen, Spiralen oder komplexe Körper vorteilhaft. Die Erfindung ist auch anwendbar bei Okkludern als Verschlusssysteme für Hohlraumverbindungen, Gefäße oder Gangsysteme.

Es ist außerdem vorteilhaft, das Implantat als Befestigungs- oder Stützvorrichtung für die temporäre Fixierung von Gewebeteilen in Form von Implantaten oder Transplantaten vorzusehen.

Zur Einstellung der Korrosionsgeschwindigkeit des Werkstoffs ist von Vorteil, wenn die Materialstärke des Werkstoffs in Abhängigkeit von der Zusammensetzung des Werkstoffs so gewählt ist, dass der Abbau- oder Korrosionsvorgang in vivo zwischen 5 Tagen und 6 Monaten, insbesondere zwischen 2 Wochen und 8 Wochen, im wesentlichen abgeschlossen ist.

Hierbei wird erreicht, dass nach einem Anwachsen des Gewebeimplantats die dann nicht mehr benötigte Fixiervorrichtung verschwindet.

Im folgenden werden verschiedene Ausführungsbeispiele der vorliegenden Erfindung gegeben.

### Beispiel 1: Gefäßstütze

Ein erfindungsgemäßer Stent wird aus einem rohrförmigen Grundkörper des metallischen und nachfolgender Bearbeitung gefertigt. Vom mechanischen Aufbau her sind derartige Stents beispielsweise aus der EP 0221570 B1 bekannt, wobei das Material jedoch ein korrosionsbeständiger Edelstahl ist.

Bei dem erfindungsgemäßen Stent nach diesem Beispiel ist das Material eine Legierung mit dem Hauptbestandteil Magnesium und gegebenenfalls den Nebenbestandteilen Lithium, Eisen, Zink und Spuren von Nickel. Die prozentuale Zusammensetzung der Magnesiumlegierung soll etwa im Bereich von 50 - 98% Magnesium, 0 - 40% Lithium, 0 - 5% Eisen und unter 5% andere Metalle liegen. Die Wandstärke der Stentstreben soll nach der Bearbeitung zwischen 50 und 100 µm betragen.

In der Praxis wird der erfindungsgemäße Stent in an sich bekannter Weise mit einem Ballonkatheter in ein krankhaft verengtes Blutgefäß eingesetzt und dort dilatiert oder als selbstexpandierender Stent freigesetzt, wobei er das Blutgefäß auf dem gewünschten Durchmesser hält. Eine ohne Stent-Implantation verbleibende Restenose (Recoil) und/oder ein durch die Dilatation induzierter Geweberiss werden wirkungsvoll behandelt. Innerhalb von 2 - 4 Wochen wird der Stent von Intimagewebe überdeckt und behält seine Stützfunktion zunächst bei. Das Blutgefäß erhält durch Gewebewachstum infolge von Eigenreparaturvorgängen im Bereich des implantierten Stents eine neue Eigenstabilität. Das Gefäßlumen wird auf einem optimalen Niveau stabilisiert. Die Wahl des Legierungsmaterials zusammen mit der gewählten Wandstärke führen andererseits dazu, dass der Stent in der Wandung des Blutgefäßes allmählich abgebaut wird und nach etwa 4 - 12 Wochen nur noch in Spuren vorliegt. Die auf Seite 2 geschilderten Nachteile eines Dauerimplantats gehen verloren.

### Beispiel 2: Verschlusssystem

Ein erfindungsgemäßes Verschlusssystem (Schirmchen) wird aus einem metallischen Skelett, an dem ein Kunststoffschirmchen befestigt ist, gefertigt. Derartige Schirmchen sind bekannt beispielsweise aus der Legierung MP35N oder Nitinol. Derartige Verschlusssysteme werden zum Verschluss von Defekten in den Herzscheidewänden verwendet. Die Wandstärke des metallischen Gerüstes beträgt um 500 mm. In der Praxis wird das Schirmchen in an sich bekannter Weise zusammengefaltet und in dem zu verschließenden Defekt freigesetzt. Innerhalb von 3 - 4 Wochen wird das Schirmchen vom körpereigenen Gewebe bedeckt und erhält durch dieses Gewebewachstum eine neue Eigenstabilität. Die Wahl des Legierungsmaterials zusammen mit der Gewebewandstärke führt dazu, dass das metallische Gerüst innerhalb von 4 Wochen bis einigen Monaten abgebaut wird und nach einem Jahr nur noch in Spuren vorliegt. Der Kunststoffanteil des Schirmchens bleibt erhalten, was aufgrund der Flexibilität des Materials unkritisch ist. Der Abbau des metallischen Anteils hat gegenüber den bekannten Schirmchen den Vorteil, dass auch bei unvorhergesehenen Belastungen z.B. bei Verkehrsunfällen keine Gefahr des Durchstoßens von Gefäßwandungen mehr besteht. Dabei wird der erfindungsgemäße Vorteil bereits erreicht, wenn zunächst durch die Degradation eine mechanische Instabilität des Gerüsts entsteht.

### Beispiel 3: Spirale zum Verschließen von Gefäßen (Okkluder)

Eine erfindungsgemäße Spirale (Coil) wird aus einem in Helixform gewickelten metallischen Material gefertigt und die Spirale vorgebogen. Der Durchmesser der Primärwicklung beträgt 0,1 - 1 mm, je nach dem zu verschließenden Gefäß. Derartige Spiralen (Coils) sind bekannt, beispielsweise aus Nitinol, Platinlegierungen oder Wolframlegierungen.

In der Praxis wird die Verschlussspirale (Coil) in an sich bekannter Weise in gestrecktem Zustand in einen Herzkatheter eingeführt und durch diesen bis zu dem zu verschließenden Gefäß vorgeschoben. Bei der Freisetzung aus dem Herzkatheter nimmt die Spirale wieder ihre alte Form an und verschließt durch ihr Lumen und ihre Thrombogenität, die durch Dacron oder andere Fasern erhöht werden kann, das zu verschließende Gefäß. Nach Thrombosierung des Gefäßes und Einwachsen von Bindegewebe erhält der Verschlussmechanismus eine neue Eigenstabilität. Die applizierte Spirale wird allmählich abgebaut, so dass nach etwa einem Jahr das implantierte Material nur noch in Spuren vorliegt.

Die insoweit genannten Ausführungsbeispiele sind mit Magnesiumlegierungen gefertigt. Toxische Wirkungen der Materialien bei den zu erwartenden Konzentrationen sind nicht bekannt.

Magnesiumlegierungen haben den Vorteil, dass durch geeignete Wahl der übrigen Legierungsbestandteile die in vivo zu erwartende Abbaugeschwindigkeit sehr genau gewählt werden kann. Außerdem ist Magnesium physiologisch sehr gut verträglich.

## Patentansprüche

1. Medizinisches Implantat aus einem durch Korrosion in vivo abbaubaren metallischen Werkstoff, wobei der Werkstoff eine Legierung ist, deren Hauptbestandteil Magnesium ist, und wobei das medizinische Implantat ein Okkluder als Verschlußsystem für Hohlraumverbindungen, Gefäße oder Gangsysteme, eine Befestigungs- oder Stützvorrichtung für die temporäre Fixierung von Gewebeimplantaten oder -transplantaten oder eine Gefäßstütze ist, wobei das Implantat einen rohrförmigen Grundkörper aufweist oder wobei das Implantat eine Spirale (Coil), ein Schirm, ein Stent, ein Drahtgeflecht, ein Clip oder ein Pfropfen ist.

2. Medizinisches Implantat nach Anspruch 1, **dadurch gekennzeichnet, daß** der Werkstoff 50 bis 98 % Magnesium, 0 bis 40 % Lithium, 0 bis 5 % Eisen und unter 5 % andere Metalle oder Seltene Erden enthält.

3. Medizinisches Implantat nach Anspruch 1, **dadurch gekennzeichnet, daß** der Werkstoff 79 bis 97 % Magnesium, 2 bis 5 % Aluminium, 0 bis 12 % Lithium und 1 bis 4 % Seltene Erden, insbesondere Cer, Lanthan, Neodym und/oder Praseodym enthält.

4. Medizinisches Implantat nach Anspruch 1, **dadurch gekennzeichnet, daß** der Werkstoff 85 bis 91 % Magnesium, 6 bis 12 % Lithium, 2 % Aluminium und 1 % Seltene Erden enthält.

5. Medizinisches Implantat nach Anspruch 1, **dadurch gekennzeichnet, daß** der Werkstoff 86 bis 97 % Magnesium, 0 bis 8 % Lithium, 2 bis 4 % Aluminium und 1 bis 2 % Seltene Erden enthält.

6. Medizinisches Implantat nach Anspruch 1, **dadurch gekennzeichnet, daß** der Werkstoff 8,5 bis 9,5 % Aluminium, 0,15 bis 0,4 % Mangan, 0,45 bis 0,9 % Zink und den Rest zu 100 % Magnesium enthält.

7. Medizinisches Implantat nach Anspruch 1, **dadurch gekennzeichnet, daß** der Werkstoff 4,5 bis 5,3 % Aluminium, 0,28 bis 0,5 % Mangan und den Rest zu 100 % Magnesium enthält.

8. Medizinisches Implantat nach Anspruch 1, **dadurch gekennzeichnet, daß** der Werkstoff 55 bis 65 % Magnesium, 30 bis 40 % Lithium und 0 bis 5 % andere Metalle und/oder Seltene Erden enthält.

9. Medizinisches Implantat nach einem der vorhergehenden Ansprüche, **dadurch gekennzeichnet, daß** das Implantat eine endoluminale Stützfunktion in Hohlorganen und/oder Gangsystemen, insbesondere Harnleitern, Gallengängen, Harnröhren, Uterus oder Bronchien, aufweist.

10. Medizinisches Implantat nach einem der vorhergehenden Ansprüche, **dadurch gekennzeichnet, daß** die Materialstärke des Werkstoffs in Abhängigkeit von der Zusammensetzung des Werkstoffs so gewählt ist, daß der Abbau- oder Korrosionsvorgang in vivo im Bereich von 5 Tagen bis zu 6 Monaten, insbesondere 2 Wochen bis 8 Wochen, abgeschlossen ist.

11. Medizinisches Implantat nach einem der vorhergehenden Ansprüche, **dadurch gekennzeichnet, daß** der Abbau- oder Korrosionsvorgang in vivo zunächst zu einer mechanischen Instabilität führt, bevor der Abbauvorgang abgeschlossen ist.

12. Medizinisches Implantat nach einem der vorhergehenden Ansprüche, **dadurch gekennzeichnet, daß** der Werkstoff Eisen und Zink in gleicher Konzentration enthält.

13. Medizinisches Implantat nach einem der vorhergehenden Ansprüche, **dadurch gekennzeichnet, daß** der Werkstoff als Nebenbestandteil ein oder mehrere Elemente aus der Gruppe enthält die folgendes umfaßt: Mn, Co, Ni, Cr, Cu, Cd, Pb, Sn, Th, Zr, Ag, Au, Pd, Pt, Re, Si, Ca, Li, Al, Zn, Fe, C, S.

## Claims

1. A medical implant made from a metallic material, which is degradable in vivo by means of corrosion, wherein the material is an alloy, the main constituent of which is magnesium, and wherein the medical implant is an occluder as a closure system for cavity connections, vessels, or ductal systems, a fastening or support device for the temporary fixation of tissue implants or transplants, or a vascular support, wherein the implant comprises a tubular base body or wherein the implant is a coil, an occluder, a stent, a wire mesh, a clip, or a plug.

2. The medical implant according to claim 1, **characterized in that** the material contains 50 to 98% magnesium, 0 to 40% lithium, 0 to 5% iron, and less than 5% other metals or rare earth metals.

3. The medical implant according to claim 1, **characterized in that** the material contains 79 to 97% magnesium, 2 to 5% aluminum, 0 to 12% lithium and 1 to 4% rare earth metals, in particular cerium, lanthanum, neodymium, and/or praseodymium.

4. The medical implant according to claim 1, **characterized in that** the material contains 85 to 91% magnesium, 6 to 12% lithium, 2% aluminum and 1% rare earth metals.

5. The medical implant according to claim 1, **characterized in that** the material contains 86 to 97% magnesium, 0 to 8% lithium, 2 to 4% aluminum and 1 to 2% rare earth metals.

6. The medical implant according to claim 1, **characterized in that** the material contains 8.5 to 9.5% aluminum, 0.15 to 0.4% manganese, 0.45 to 0.9% zinc and magnesium for the rest up to 100%.

7. The medical implant according to claim 1, **characterized in that** the material contains 4.5 to 5.3% aluminum, 0.28 to 5% manganese, and magnesium for the rest up to 100%.

8. The medical implant according to claim 1, **characterized in that** the material contains 55 to 65 % magnesium, 30 to 40% lithium, 0 to 5% other metals and/or rare earth metals.

9. The medical implant according to any one of the preceding claims, **characterized in that** the implant has an endoluminal support function in hollow organs, and/or ductal systems, in particular ureters, bile ducts, urethras, the uterus or bronchia.

10. The medical implant according to any one of the preceding claims, **characterized in that** the material strength of the material is selected depending on the composition of the material such that the degradation or corrosion process, in vivo, is substantially closed in the range of 5 days up to 6 months, in particular 2 weeks to 8 weeks.

11. The medical implant according to any one of the preceding claims, **characterized in that** the degradation or corrosion process, in vivo, initially results in a mechanical instability before the degradation process is concluded.

12. The medical implant according to any one of the preceding claims, **characterized in that** the material contains iron and zinc in an identical concentration.

13. The medical implant according to any one of the preceding claims, **characterized in that** the material contains, as the minor constituent, one or more elements from the group comprising the following: Mn, Co, Ni, Cr, Cu, Cd, Pb, Sn, Th, Zr, Ag, Au, Pd, Pt, Re, Si, Ca, Li, Al, Zn, Fe, C, S.

## Revendications

1. Implant médical à base d'un matériau métallique dégradable in vivo par corrosion, dans lequel le matériau est un alliage dont le constituant principal est du magnésium, et dans lequel l'implant médical est un occludeur servant de système de fermeture pour des liaisons entre des cavités, des vaisseaux ou des systèmes de canaux, un dispositif de fixation ou de soutien pour la fixation temporaire d'implant tissulaires ou de greffons tissulaires, ou un support de vaisseau, dans lequel l'implant présente un corps de base en forme de tube ou dans lequel l'implant est une spirale (coil), un filtre parapluie, un stent, un treillis métallique, un clip ou un bouchon.

2. Implant médical selon la revendication 1, **caractérisé en ce que** le matériau contient 50 à 98 % de magnésium, 0 à 40 % de lithium, 0 à 5 % de fer et moins de 5 % d'autres métaux ou d'alcalino-terreux.

3. Implant médical selon la revendication 1, **caractérisée en ce que** le matériau contient de 79 à 97 % de magnésium, 2 à 5 % d'aluminium, 0 à 12 % de lithium et 1 à 4 % de terres rares, notamment de cérium, de lanthane, de néodyme et/ou de praséodyme.

4. Implant médical selon la revendication 1, **caractérisé en ce que** le matériau contient 85 à 91 % de magnésium, 6 à 12 % de lithium, 2 % d'aluminium et 1 % de terres rares.

5. Implant médical selon la revendication 1, **caractérisé en ce que** le matériau contient 86 à 97 % de magnésium, 0 à 8 % de lithium, 2 à 4 % d'aluminium et 1 à 2 % de terres rares.

6. Implant médical selon la revendication 1, **caractérisé en ce que** le matériau contient 8,5 à 9,5% d'aluminium, 0,15 à 0,4% de manganèse, 0,45 à 0,9 % de zinc et le complément à 100 % de magnésium.

7. Implant médical selon la revendication 1, **caractérisé en ce que** le matériau contient 4,5 à 5,3 % d'aluminium, 0,28 à 0,5 % de manganèse et le complément à 100 % de magnésium.

8. Implant médical selon la revendication 1, **caractérisé en ce que** le matériau contient 55 à 65 % de magnésium, 30 à 40 % de lithium et 0 à 5 % d'autres métaux et/ou de terres rares.

9. Implant médical selon l'une des revendications précédentes, **caractérisé en ce que** l'implant présente une fonction de support endoluminal dans des organes creux et/ou dans systèmes de canaux, notamment les uretères, les canaux vésiculaires, les urètres, l'utérus ou les bronches.

10. Implant médical selon l'une des revendications précédentes, **caractérisé en ce que** la résistance du matériau est choisie en fonction de la composition du matériau de telle sorte que le processus de dégradation ou de corrosion in vivo est terminé dans un intervalle de 5 jours jusqu'à 6 mois, notamment, de 2 semaines à 8 semaines.

11. Implant médical selon l'une des revendications précédentes, **caractérisé en ce que** le processus de dégradation ou de corrosion in vivo mène d'abord à une instabilité mécanique, avant que le processus de dégradation ne soit terminé.

12. Implant médical selon l'une des revendications précédentes, **caractérisé en ce que** le matériau contient du fer et du zinc à des concentrations égales.

13. Implant médical selon l'une des revendications précédentes, **caractérisé en ce que** le matériau contient en tant que constituant secondaire un ou plusieurs éléments faisant partie du groupe qui est composé des éléments suivants : Mn, Co, Ni, Cr, Cu, Cd, Pb, Sn, Th, Zr, Ag, Au, Pd, Pt, Re, Si, Ca, Li, Al,, Zn, Fe, C, S.
